(19) **Europäisches Patentamt European Patent Office Office européen des brevets**

(11) **EP 1 903 499 A2**

(12) **EUROPEAN PATENT APPLICATION**

(43) Date of publication:
26.03.2008 Bulletin 2008/13

(51) Int Cl.:
**G06T 5/00** $^{(2006.01)}$

(21) Application number: 07116212.7

(22) Date of filing: 12.09.2007

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: 22.09.2006 JP 2006257762

(71) Applicant: Konica Minolta Medical & Graphic, Inc.
Hino-shi,
Tokyo 191-8511 (JP)

(72) Inventors:
• **Kido, Kazuhiro**
**Tokyo 192-8505 (JP)**
• **Oishi, Atsushi**
**Tokyo 191-8511 (JP)**
• **Umeda, Toshikazu**
**Tokyo 192-8505 (JP)**

(74) Representative: **Henkel, Feiler & Hänzel**
**Patentanwälte**
**Maximiliansplatz 21**
**80333 München (DE)**

(54) **Radiological image capturing system and radiological image capturing method**

(57)    There is described a radiological image capturing system, which makes it possible to obtain an accurate radiological image, irrespective of the position of the diagnosis object in the thickness direction of the subject. The system includes a radiographing apparatus that output radiological image data, based on the radial rays detected by the image detecting device, an image processing apparatus that applies a degraded image restoration processing to the radiological image data and a fixing member onto which the subject is fixed. The subject is positioned at such a position that a ratio of blurs between the first radiological image radiographed at a first position and the second radiological image radiographed at a second position, which is apart from the image detecting device by a distance farther than that of the first position, is reduced to a value to such an extent that a blur difference between them can be ignored.

FIG. 2

## EP 1 903 499 A2

**Description**

This application is based on Japanese Patent Application No. 2006-257762 filed on September 22, 2006 with Japan Patent Office, the entire content of which is hereby incorporated by reference.

**BACKGROUND OF THE INVENTION**

The present invention relates to a radiological image capturing system and a radiological image capturing method for capturing a radiological image of a subject by irradiating radial rays onto the subject.

In recent years, efficiencies of the radial ray irradiating apparatus and the radial ray detecting device have been improved, and it has become possible to rapidly recognize a subtle lesion of a certain illness from the radiological image in conjunction with the CAD (Computer Aided Diagnosis). Further, with respect to the radiological image capturing system, it has been required that a high quality and high resolution radiological image, which makes it possible to make high accurate diagnosis of such the subtle lesion, can be obtained, even if a small amount of radiation exposure dose of radial ray is used for capturing the radiological image.

In this connection, blurs caused by the scattered radial rays, etc., generated inside the body of the subject, generally occur in the radiological image. Accordingly, to eliminate such the blurs in order to obtain the high quality and high resolution radiological image, there has been proposed a radiological image capturing system that applies a restoration processing, which employs the Point Spread Function (hereinafter, also referred to as the PSF) as the image restoration parameter, to the image data representing the radiological image captured.

For instance, Patent Document 1 (JP-2509181, Japanese Patent Gazette) sets forth a radiological image capturing system that includes the steps of: compensating for the Point Spread Function representing the distribution of the X rays scattered on the surface of the detector with respect to the incident X rays penetrated into the subject, corresponding to the radiographing conditions of the X ray original image, so as to output the filter coefficients; finding the scattered X ray image by convoluting the filter coefficients and the X ray original image with each other; and subtracting the scattered X ray image from the X ray original image so as to directly obtain the X ray image.

Further, Patent Document 2 (JP-3423828, Japanese Patent Gazette) sets forth another radiological image capturing system that includes the steps of: finding the intensity distribution function of the scattered light from the product of the intensity ratio of the scattered light and the Point Spread Function of the scattered light in the state of placing no subject, so as to calculate the image of the scattered light component; finding the intensity distribution function of the scattered rays from the product of the Point Spread Function of the scattered rays and the intensity ratio, derived from the subject image and the radiographing condition, in the state of placing the human dummy subject having a uniform thickness, so as to calculate the image of the scattered ray component; and subtracting the image of the scattered light component and the image of the scattered ray component from the image of the above subject, so as to restore the X ray image in the degraded image restoration mode.

However, in the real diagnosing practice, the doctor should inspect various kinds of lesions and organs, which would vary from patients to patients. Accordingly, when a lesion or an organ, being a diagnosis objects, exists at a deviated position in the body of the patients, sometimes, it has been impossible to restore the accurate X ray image from the blurred image in the degraded image restoration mode, even if the degraded image restoration processing is applied to the X ray image data by employing the predetermined Point Spread Function corresponding to the radiographing conditions as the image restoration parameter, as set forth in Patent Document 1 or Patent Document 2.

Concretely speaking, when positions of a specific diagnosis object for various patients in a thickness direction of the subject are different from each other, the point spread distribution of the X rays penetrated through the diagnosis object also varies with the various patients. Accordingly, since the image restoration parameter for restoring the X ray image from the blurred image in the degraded image restoration mode also varies with the various patients, there has been a problem that it is impossible to restore the accurate X ray image from the blurred image in the degraded image restoration mode, which employs a single image restoration parameter.

On the other hand, there has been another problem that, if the degraded image restoration processing is conducted by employing one of plural image restoration parameters, which vary with the positions of the diagnosis object in the thickness direction of the subject, the contents of such the processing become complicated, resulting in increase of the cost, and further, it is impossible to improve the efficiency of the processing.

**SUMMARY OF THE INVENTION**

To overcome the abovementioned drawbacks in conventional radiological image capturing systems and methods, it is one of objects of the present invention to provide a radiological image capturing system and a radiological image capturing method, which makes it possible to obtain a radiological image, being more accurate than ever, by employing a simple and easy processing, irrespective of the position of the diagnosis object in the thickness direction

2

of the subject.

Accordingly, at least one of the objects of the present invention can be attained by any one of the radiological image capturing systems and the radiological image capturing methods described as follows.

(1) According to a radiological image capturing system reflecting an aspect of the present invention, the radiological image capturing system comprises: a radiographing apparatus that is provided with a radial ray emitting source to emit and irradiate radial rays onto a subject and an image detecting device to detect radial rays penetrated through the subject, so as to output radiological image data based on the radial rays detected by the image detecting device; and an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and a fixing member to fix the subject onto a predetermined position; and wherein formula (1) indicated as follow is fulfilled:

$$R2 \rangle \frac{A}{R1} \times Log_{10}D \quad \cdots (1)$$

where R1 (m): distance between the radial ray emitting source and the predetermined position of the subject fixed onto the fixing member,

R2 (m): distance between the predetermined position of the subject and the image detecting device,

D ($\mu$m): focusing aperture of the radial ray emitting source, and

A: constant value equal to or greater than 0.05.

(2) According to another aspect of the present invention, in the radiological image capturing system recited in item 1, when a first point spread distribution of first radiological image data, to be detected by the image detecting device at a first diagnosis object position of the subject, is defined as $\delta 1$, while a second point spread distribution of second radiological image data, to be detected by the image detecting device at a second diagnosis object position of the subject, which is apart from the image detecting device by a distance farther than that of the first diagnosis object position, is defined as $\delta 2$, the first point spread distribution $\delta 1$ and the second point spread distribution $\delta 2$ fulfill a relationship indicated as follow:

$$\delta 1 / \delta 2 \leq 5 .$$

(3) According to still another aspect of the present invention, in the radiological image capturing system recited in item 2, a gap distance between the first diagnosis object position and the second diagnosis object position is equal to or shorter than 300 mm in an irradiating direction of the radial rays.

(4) According to yet another aspect of the present invention, in the image forming apparatus recited in any one of items 1 - 3, the image processing section is further provided with an image restoration parameter creating section to create the image restoration parameter based on reference radiological image data of the subject radiographed by the radiographing apparatus.

(5) According to a method for capturing radiological image, reflecting another aspect of the present invention, the method for capturing radiological image, to be employed in a radiological image capturing system, which includes: a radiographing apparatus that is provided with a radial ray emitting source to emit and irradiate radial rays onto a subject and an image detecting device to detect radial rays penetrated through the subject, so as to output radiological image data based on the radial rays detected by the image detecting device; an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and a fixing member to fix the subject onto a predetermined position, comprises:

fixing the subject onto the fixing member, so as to position the subject at the predetermined position, which fulfills formula (1) indicated as follow:

$$R2 \rangle \frac{A}{R1} \times Log_{10}D \quad \cdots (1)$$

where R1 (m): distance between the radial ray emitting source and the predetermined position of the subject fixed onto the fixing member,

R2 (m): distance between the predetermined position of the subject and the image detecting device,

D ($\mu$m): focusing aperture of the radial ray emitting source, and

A: constant value equal to or greater than 0.05.

(6) According to still another aspect of the present invention, in the method recited in item 5, when a first point spread distribution of first radiological image data, to be detected by the image detecting device at a first diagnosis object position of the subject, is defined as $\delta1$, while a second point spread distribution of second radiological image data, to be detected by the image detecting device at a second diagnosis object position of the subject, which is apart from the image detecting device by a distance farther than that of the first diagnosis object position, is defined as $\delta2$, the first point spread distribution $\delta1$ and the second point spread distribution $\delta2$ fulfill a relationship indicated as follow:

$$\delta1 \ / \ \delta2 \ \leq \ 5 \ .$$

(7) According to still another aspect of the present invention, in the method recited in item 6, a gap distance between the first diagnosis object position and the second diagnosis object position is equal to or shorter than 300 mm in an irradiating direction of the radial rays.

(8) According to yet another aspect of the present invention, in the method recited in any one of items 5 - 7, the method further comprises: creating the image restoration parameter based on reference radiological image data of the subject radiographed by the radiographing apparatus.

(9) According to a radiological image capturing system reflecting still another aspect of the present invention, the radiological image capturing system comprises: a radiographing apparatus that is provided with a radial ray emitting source to emit and irradiate radial rays onto a subject and an image detecting device to detect radial rays penetrated through the subject, so as to output radiological image data based on the radial rays detected by the image detecting device; and an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and a fixing member onto which the subject is fixed; and wherein the fixing member is employed for positioning the subject at such a position that a ratio of blurs between a first radiological image radiographed at a first diagnosis object position of the subject and a second radiological image radiographed at a second diagnosis object position of the subject, which is apart from the image detecting device by a distance farther than that of the first diagnosis object position, is reduced to a value to such an extent that a difference of blurs between the first radiological image and the second radiological image can be ignored.

(10) According to still another aspect of the present invention, in the radiological image capturing system recited in item 9, the subject is positioned so as to fulfill formula (1) indicated as follow:

$$R2 \rangle \frac{A}{R1} \times Log_{10}D \quad \cdots (1)$$

where R1 (m): distance between the radial ray emitting source and the predetermined position of the subject fixed onto the fixing member,

R2 (m): distance between the predetermined position of the subject and the image detecting device,

D ($\mu$m): focusing aperture of the radial ray emitting source, and

A: constant value equal to or greater than 0.05.

(11) According to still another aspect of the present invention, in the radiological image capturing system recited in item 9 or 10, when a first point spread distribution of first radiological image data, to be detected by the image detecting device at the first diagnosis object position of the subject, is defined as $\delta1$, while a second point spread distribution of second radiological image data, to be detected by the image detecting device at the second diagnosis object position of the subject is defined as δ2, the first point spread distribution δ1 and the second point spread distribution δ2 fulfill a relationship indicated as follow:

$$\delta1 \ / \ \delta2 \leq 5 \ .$$

(12) According to still another aspect of the present invention, in the radiological image capturing system recited in item 11, a gap distance between the first diagnosis object position and the second diagnosis object position is equal to or shorter than 300 mm in an irradiating direction of the radial rays.

(13) According to yet another aspect of the present invention, in the radiological image capturing system recited in any one of items 9 - 12, the image processing section is further provided with an image restoration parameter creating section to create the image restoration parameter based on reference radiological image data of the subject radiographed by the radiographing apparatus.

(14) According to a method for capturing radiological image, reflecting still another aspect of the present invention, the method for capturing radiological image, to be employed in a radiological image capturing system, which includes: a radiographing apparatus that is provided with a radial ray emitting source to emit and irradiate radial rays onto a subject and an image detecting device to detect radial rays penetrated through the subject, so as to output radiological image data based on the radial rays detected by the image detecting device; an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and a fixing member onto which the subject is fixed, comprises: fixing the subject onto the fixing member, so as to position the subject at such a position that a ratio of blurs between a first radiological image radiographed at a first diagnosis object position of the subject and a second radiological image radiographed at a second diagnosis object position of the subject, which is apart from the image detecting device by a distance farther than that of the first diagnosis object position, is reduced to a value to such an extent that a difference of blurs between the first radiological image and the second radiological image can be ignored.

(15) According to still another aspect of the present invention, in the method recited in item 14, the subject is positioned so as to fulfill formula (1) indicated as follow:

$$R2 > \frac{A}{R1} \times Log_{10}D \quad \cdots(1)$$

where R1 (m): distance between the radial ray emitting source and the predetermined position of the subject fixed onto the fixing member,

R2 (m): distance between the predetermined position of the subject and the image detecting device,

D (μm): focusing aperture of the radial ray emitting source, and

A: constant value equal to or greater than 0.05.

(16) According to still another aspect of the present invention, in the method recited in item 14 or 15, when a first point spread distribution of first radiological image data, to be detected by the image detecting device at a first diagnosis object position of the subject, is defined as δ1, while a second point spread distribution of second radiological image data, to be detected by the image detecting device at a second diagnosis object position of the subject, which is apart from the image detecting device by a distance farther than that of the first diagnosis object position, is defined as δ2, the first point spread distribution δ1 and the second point spread distribution δ2 fulfill a relationship indicated as follow:

$$\delta1 \ / \ \delta2 \leq 5 \ .$$

(17) According to still another aspect of the present invention, in the method recited in item 16, a gap distance between the first diagnosis object position and the second diagnosis object position is equal to or shorter than 300 mm in an irradiating direction of the radial rays.

(18) According to yet another aspect of the present invention, in the method recited in any one of items 14 - 17, the method further comprises: creating the image restoration parameter based on reference radiological image data of the subject radiographed by the radiographing apparatus.

## BRIEF DESCRIPTION OF THE DRAWINGS

Embodiments will now be described, by way of example only, with reference to the accompanying drawings which are meant to be exemplary, not limiting, and wherein like elements are numbered alike in several Figures, in which:

Fig. 1 shows a schematic diagram of an overall configuration of a radiological image capturing system embodied in the present invention;
Fig. 2 shows a plane view schematically indicating a positional relationship between an X ray tube, a subject and an image detecting device in a radiographing apparatus, embodied in the present invention;
Fig. 3 shows a graph indicating transitions of ratio of blurs, corresponding to variation of each diagnosis object position;
Fig. 4 shows a graph indicating transitions of ratio of blurs, corresponding to variation of each diagnosis object position;
Fig. 5 shows a controlling configuration of a radiological image capturing system embodied in the present invention;
Fig. 6 shows a plane view indicating a structure of a shielding member embodied in the present invention;
Fig. 7 shows an example of a Point Spread Function employed in the present invention; and
Fig. 8 shows a flowchart indicating an image processing operation to be conducted by an image processing section embodied in the present invention.

## DETAILED DESCRIPTION OF THE PREFERRED EMBODIMENT

Referring to the drawings, the radiological image capturing system, embodied in the present invention, will be detailed in the following. However, the scope of the present invention is not limited to the examples illustrated as follows.

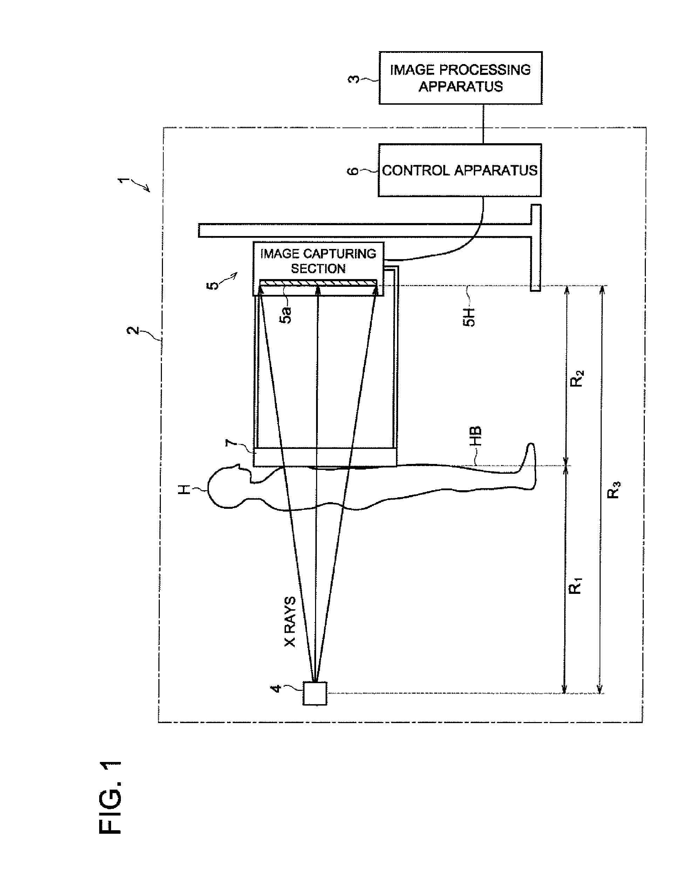

Fig. 1 shows a schematic diagram of an overall configuration of a radiological image capturing system 1 embodied in the present invention.

As shown in Fig. 1, the radiological image capturing system 1 is constituted by a radiographing apparatus 2 to capture an X ray image of a subject H and an image processing apparatus 3 to conduct various kinds of image processing, in order to capture the X ray image by irradiating X rays onto the subject H and to apply the image processing, such as a degraded image restoration processing, etc., to the X ray image captured by the radiographing apparatus 2. Incidentally, the scope of the radial rays is not limited to the X rays, but any one of gamma rays, electron rays, etc., is also applicable in the present invention.

The radiographing apparatus 2 is provided with an X ray tube 4, serving as a source of radial rays, an image capturing section 5 and a control apparatus 6, and is so constituted that the X ray image of the subject H, fixed at a predetermined position by a fixing member 7, can be captured. In this connection, the fixing member 7 is made of a material, such as a resin, etc., through which the radial rays can penetrate.

The X ray tube 4 is equipped at a position located in the backside space of the subject H, and generates X rays having a predetermined focusing aperture so as to irradiate the radial rays towards the subject H. In this connection, the larger the focusing aperture of the X ray tube 4 is, the greater an amount of the X rays to be irradiated within a fixed time interval becomes.

The image capturing section 5 is equipped at a position located in a front side space of the subject H, in such a manner that the height of the image capturing section 5 can be adjusted according to a current photographing portion, serving as a diagnosis object. Further, the image capturing section 5 incorporates an image detecting device 5a.

The image detecting device 5a is configured to detect the X rays irradiated onto its detecting surface. A stimulable phosphor plate, a FPD (Flat Panel Detector), etc., each of which is capable of absorbing and storing energy of the irradiated X rays, can be applied for the image detecting device 5a. When employing the stimulable phosphor plate, the image capturing section 5 is provided with a reading section (not shown in the drawings) that irradiates an excitation light, such as a laser beam, etc., onto the stimulable phosphor plate so as to electro-photographically convert the stimulated light emitted from the stimulable phosphor plate to image signals. This image signals (analogue signals) generated by the reading section are outputted to the control apparatus 6.

On the other hand, the FPD is configured by aligning conversion elements, each of which generates an electric signal having an intensity corresponding to the amount of the incident X ray, into a matrix pattern, and differs from the stimulable phosphor plate on the point that the electric signals are directly generated within the FPD. When employing the FPD, an analogue-to-digital converter (not shown in the drawings) converts the analogue electric signals, generated in the FPD, to digital image data, which are outputted to the control apparatus 6.

The image detecting device 5a, embodied in the present invention, is positioned so as to fulfill the condition represented by the formula (1) shown as follow.

$$R2 \rangle \frac{A}{R1} \times Log_{10} D \quad \cdots(1)$$

Where R1: distance between the X ray tube 4 and the predetermined position of the subject H fixed by the fixing member 7, for instance, a position at which the subject H contacts the fixing member 7 (subject reference position HB),

R2: distance between the subject reference position HB and a detecting surface 5H of the image detecting device 5a,

D ($\mu$m) : focusing aperture of the X ray tube 4, and

A: constant value equal to or greater than 0.05.

According to the above, the system is so constituted that the accurate X ray radiological image can be obtained by applying the degraded image restoration processing, which employs a single image restoration parameter, irrespective of the position of the diagnosis object in the thickness direction of the subject H. In this connection, although the upper limits of R1 and R2 can be determined as needed, corresponding to the installation place of the radiographing apparatus 2, etc., in the present embodiment, R1 and R2 are determined so as to fulfill the following relationship:

```
R3 = R1 + R2 ≤ 3 m (meters), preferably R3 ≤ 1.5 m
```

where R3: distance between the X ray tube 4 and the detecting surface 5H.

Herein, the reason why the accurate X ray radiological image can be obtained by applying the degraded image restoration processing, which employs a single image restoration parameter, irrespective of the position of the diagnosis object in the thickness direction of the subject H, when the image detecting device 5a and the fixing member 7 are disposed at such positions that fulfills the formula (1) mentioned in the above, will be detailed in the following.

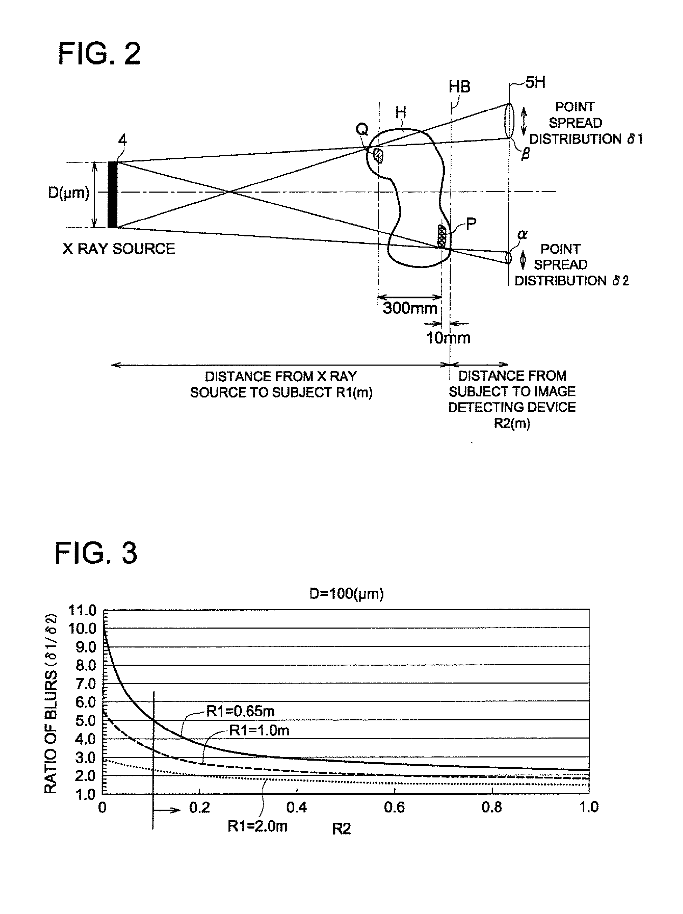

Fig. 2 shows a plane view schematically indicating the positional relationship between the X ray tube 4, the subject H and the image detecting device 5a in the radiographing apparatus 2 shown in Fig. 1. As shown in Fig. 2, the subject H contacts the fixing member 7 at the subject reference position HB being apart from the detecting surface 5H of the image detecting device 5a.

Hereinafter in the present invention, it is assumed that a gap distance between a first diagnosis object position and a second diagnosis object position, which is apart from the image detecting device 5a by a distance farther than that of the first diagnosis object position, is equal to or shorter than 300 mm. In the present embodiment shown in Fig. 2, a position deviating toward the X ray tube 4 from the subject reference position HB by 10 mm is defined as a diagnosis object position P, while another position deviating toward the X ray tube 4 from the subject reference position HB by 310 mm is defined as a diagnosis object position Q. Assuming the case of capturing the X ray radiological image of the front breast image of a human body, the diagnosis object position P is made to deviate from the subject reference position HB by 10 mm, while the gap distance between the diagnosis object position P and the diagnosis object position Q is established at 300 mm in the present embodiment.

When diagnosis objects, dimension of which are the same, are respectively disposed at the diagnosis object position P and the diagnosis object position Q, as shown in Fig. 2, a radiological image $\alpha$ having a point spread distribution $\delta2$ in regard to the diagnosis object disposed at the diagnosis object position P and a radiological image $\beta$ having a point spread distribution $\delta1$ in regard to the diagnosis object disposed at the diagnosis object position Q are detected on the image detecting device 5a, respectively.

In this connection, when the positions of the diagnosis objects in the thickness direction of the subject H are different from each other, blurring degrees of the X ray radiological images caused by the diverging effect of the X rays irradiated from the X ray tube 4 are also different from each other. In the example shown in Fig. 2, the value of the point spread distribution $\delta1$ of the radiological image $\beta$ formed by X rays passing through the diagnosis object position Q is relatively large, while the value of the point spread distribution $\delta2$ of the radiological image $\alpha$ formed by X rays passing through the diagnosis object position P is relatively small. As mentioned in the above, in the case that the values of the point spread distributions $\delta1$ and $\delta2$ at the diagnosis object position Q and the diagnosis object position P are different from each other, and the image restoration parameters to be respectively employed for the degraded image restoration processing for the X ray radiological images of the diagnosis object position Q and the diagnosis object position P are different from each other, conventionally, it has been merely possible to accurately restore any one of the X ray radiological images of the diagnosis object position Q and the diagnosis object position P in the degraded image restoration mode, when only a single image restoration parameter is employed.

Fig. 3 shows a graph indicating transitions of ratio of blurs between the point spread distribution $\delta1$ of the

radiological image β and the point spread distribution δ2 of the radiological image α, corresponding to the variation of R2 in the example shown in Fig. 2. Incidentally, the focusing aperture D of the X ray tube 4 is set at 100 μm, while the value of R1 is sequentially changed to 0.65, 1.0 and 2.0 m (meters).

As shown in Fig. 3, it is revealed that the smaller the values of R1 and R2 become, the larger the ratio of blurs of the radiological image α and the radiological image β become. In this connection, it has been empirically acknowledged that, when the ratio of blurs (= δ1 / δ2) is equal to or smaller than 5.0, differences between blurs are sufficiently small to such an extent that the differences between blurs can be ignored, and accordingly, it is practically no problem to apply the degraded image restoration processing, in which only a single image restoration parameter is employed, to the radiological images concerned. In the present embodiment, it is established that the ratio of blurs (= δ1 / δ2) is equal to or smaller than 5.0, under the definition that the point spread distribution of the X ray radiological image located at the diagnosis object position P is δ2, while the other point spread distribution of the X ray radiological image located at the diagnosis object position Q, which is apart from the image detecting device 5a by a distance farther than that of the diagnosis object position P, is δ1. Further, it is more preferable that the ratio of blurs (= δ1 / δ2) is equal to or smaller than 2.5.

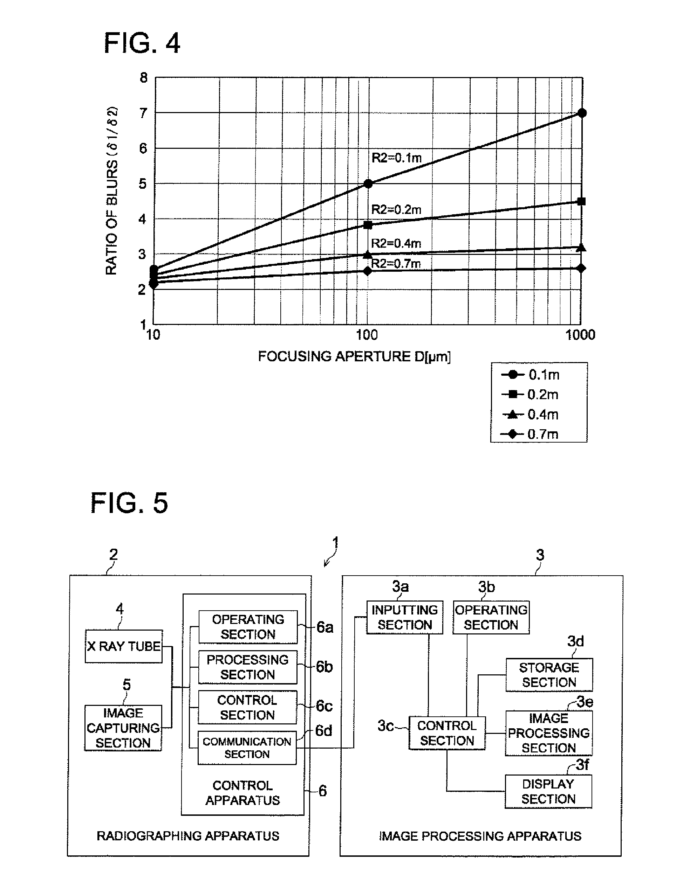

When the value of R1 is fixed at 0.65 m, Fig. 4 shows a graph indicating transitions of ratio of blurs between the point spread distribution δ1 of the radiological image β and the point spread distribution δ2 of the radiological image α, corresponding to the variation of the focusing aperture D, with respect to different values of R2, which represents a distance from the subject reference position HB to the detecting surface 5H. As shown in Fig. 4, it is revealed that ratio of blurs between the X ray radiological images of the radiological image α and the radiological image β varies in substantially a linear relationship with the common logarithms of the focusing aperture D.

Accordingly, in the present invention, based on the relationships between the ratios of blurs and the values of R1, R2 and the common logarithms of the focusing aperture D, the difference between blurs is set at a small value to such a extent that the difference between blurs of the X ray radiological images can be ignored by fulfilling the formula (1). According to the above, it becomes possible that the accurate X ray radiological image can be obtained by applying the degraded image restoration processing, which employs a single image restoration parameter, irrespective of the position of the diagnosis object in the thickness direction of the subject H. In this connection, when the constant value A is set at a value being equal to or greater than 2.0, since it becomes possible to reduce the ratio of blurs, indicated in the example shown in Fig. 2, to a value being equal to or smaller than 2.5, it becomes possible to apply the degraded image restoration processing, in which only a single image restoration parameter is employed, to the radiological image concerned without causing any practical problem, even if the gap distance between the diagnosis object position P and the diagnosis object position Q is equal to or greater than 300 mm, as in the case of radiographing the breast section from the side surface of the human body.

Now, returning to Fig. 1, the X ray tube 4 and the image capturing section 5 are electrically coupled to the control apparatus 6. Further, the image processing apparatus 3 is also electrically coupled to the control apparatus 6.

Next, referring to Fig. 5, the controlling configuration of the radiological image capturing system 1 will be detailed in the following.

As shown in Fig. 5, the radiological image capturing system 1 is constituted by the radiographing apparatus 2 and the image processing apparatus 3, while the radiographing apparatus 2 is provided with the control apparatus 6. The control apparatus 6 is electrically coupled to each of the X ray tube 4 and the image capturing section 5, and is provided with an operating section 6a to conduct controlling operations of the X ray tube 4 and the image capturing section 5, a processing section 6b to conduct various kinds of signal processing and data processing operations, such as a data conversion processing for converting analogue image signals to digital image data, etc., a control section 6c to concentrically control each of the sections of the radiographing apparatus 2 and a communication section 6d to conduct communicating operations with the other external apparatuses.

Further, the image processing apparatus 3 is provided with an inputting section 3a to input the image data (either the analogue image signals or the digital image data) read by the image detecting device 5a of the image capturing section 5, an operating section 3b to input various kinds of instructions, a control section 3c to control each of the structural sections of the image processing apparatus 3, a storage section 3d to store various kinds of control data and image data, an image processing section 3e to apply image processing to the image data based on the control data stored in the storage section 3d and a display section 3f to display an image based on the processed image data to which the image processing is already applied by the image processing section 3e.

The inputting section 3a is electrically coupled to the communication section 6d of the radiographing apparatus 2, so as to input the image data read by the image detecting device 5a.

The operating section 3b includes a mouse, a keyboard or an operation panel employing a touch panel method, etc., which are generally well-known parts, so as to make it possible for the user to input instructions in regard to the image processing operations.

The control section 3c includes a CPU (Central Processing Unit), a RAM (Random Access Memory), etc., and reads out various kinds of controlling programs stored in the storage section 3d in advance and develops them into the RAM, so as to conducts various kinds of arithmetic calculations and concentrically control each of the structural sections by executing the abovementioned controlling programs.

Further, the control section 3c serves as an image restoration parameter creating section that conducts a calculation processing of the Point Spread Function (PSF) to be stored in the storage section 3d, based on a reference radiological image of the subject radiographed by the radiographing apparatus 2, namely, an image restoration parameter creation processing. Hereinafter, the Point Spread Function (PSF) is defined as a function to be employed in the degraded image restoration processing in regard to the blurred image. Further, the reference radiological image is defined as such a radiological image that is located at a predetermined position in the thickness direction of the subject H and is employed in the calculating operation of the Point Spread Function (PSF). The calculating operation of the PSF is implemented at a certain appropriate time before radiographing the subject (for instance, at the time when installing the apparatus, the time when conducting the maintenance operations, the time when changing the X ray tube 4 or the inputting section 3a, etc.).

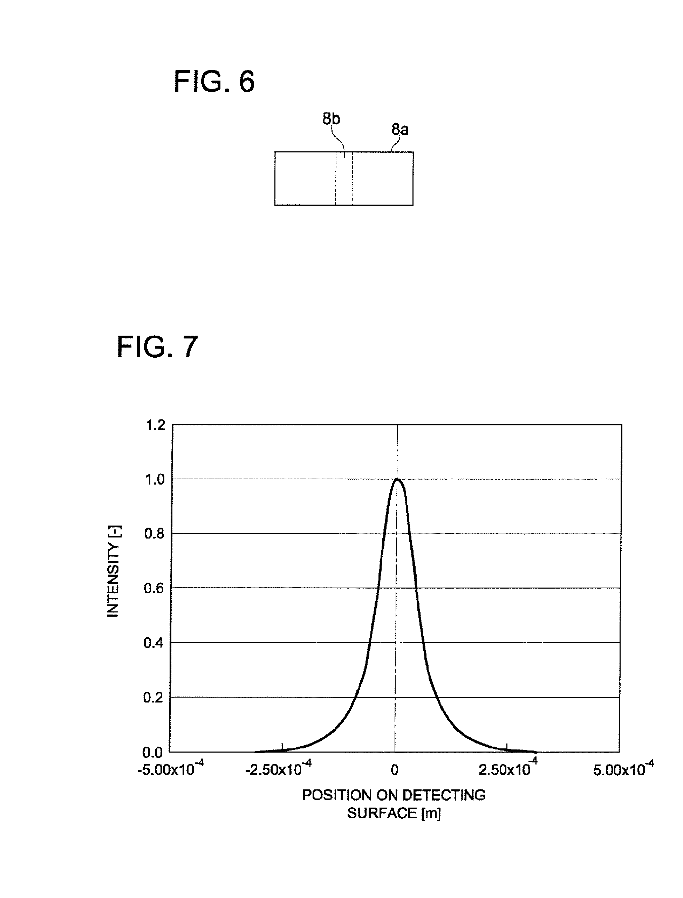

Further, the calculating operation of the PSF is implemented, for instance, in such a state that a plate member 8a (shading member) on which a through hole 8b is formed at a predetermined position, as shown in Fig. 6, is equipped in the radiographing apparatus 2. For instance, the plate member 8a, being applicable to the above, is made of a radial ray shading material, such as a lead, etc., having the through hole 8b formed at a center of the plate, as shown in Fig. 6. In the example shown in Fig. 6, the thickness of the plate member 8a is set at a value in a range of 0.01 - 0.1 mm, while the diameter of the through hole 8b is set at a value in a range of several - 50 $\mu$m. Receiving an instruction signal for commencing the PSF creating operation from the operating section 3b, the control section 3c controls the X ray tube 4 and the image capturing section 5 of the radiographing apparatus 2 so as to radiograph the through hole 8b of the plate member 8a. Further, when the X ray radiological image data acquired by the image capturing section 5 are inputted into the inputting section 3a as the reference radiological image data, the control section 3c creates the luminance distribution from the reference radiological image data, so as to create a function being approximate to a linear shape of the luminance distribution. This function created in the above is the Point Spread Function (PSF) at each diagnosis object position of the diagnosis object concerned. The created PSF is stored in the storage section 3d as the image restoration parameter.

For instance, the PSF is a distribution function shown in Fig. 7. In Fig. 7, the horizontal axis represents deviation amounts on the detecting surface 5H of the image detecting device 5a when the center position of the through hole 8b is set at zero, while the vertical axis represents the PSF when a maximum intensity of the radial rays, to be detected at the deviation amount of zero, is set at 1.0. In this connection, the PSF shown in Fig. 7 is calculated on the basis of the reference radiological image data on the conditions that the focusing aperture D of the X ray tube 4 is set at 500 $\mu$m, the distance from the X ray tube 4 to the image detecting device 5a is set at 900 mm and the diameter of the through hole 8b set at 10 $\mu$m.

Further, although the PSF only corresponding to the blurs caused by the focusing aperture D is employed in the present embodiment, it is also applicable that the PSF including other blurs caused by the scattered radial rays, which are scattered within the subject, in addition to the blurs caused by the focusing aperture D, is employed for the degraded image restoration processing.

The storage section 3d stores various kinds of programs to be executed by the control section 3c, image processing programs to be executed by the image processing section 3e, parameters and data necessary for executing the above programs, etc. in it. Further, the storage section 3d employed in the present embodiment also stores the X ray radiological image data representing the radiological image captured by the radiographing apparatus 2 with the radiographing conditions, and, for instance, the PSF as the image restoration parameter, in it.

The image processing section 3e applies the degraded image restoration processing, which employs a predetermined PSF as the image restoration parameter, to the X ray radiological image data representing the radiological image captured by the radiographing apparatus 2. Concretely speaking, the image processing section 3e reads out the predetermined PSF, correlated to the radiographing conditions, from the storage section 3d, so as to create a degradation restored image from the image restoration parameter based on the PSF concerned and the image data stored in the storage section 3d. At this occasion, by disposing the X ray tube 4, the image detecting device 5a and the fixing member 7 at such the positions that fulfill the aforementioned formula (1), since the differences between blurs become small to such the extent that the differences between blurs can be ignored, the accurate X ray radiological image can be obtained by applying the degraded image restoration processing, which employs a single image restoration parameter, irrespective of the position of the diagnosis object in the thickness direction of the subject H. In addition to the above, the image processing section 3e also conducts other kind of image processing, such as a gradation correction processing, etc.

The degraded image restoration processing conducted by the image processing section 3e is to acquire the degradation restored image by deconvoluting the X ray radiological image data inputted from the inputting section 3a with the PSF serving as the image restoration parameter. Concretely speaking, the image processing section 3e applies the Fourier Transform processing to the X ray radiological image data inputted from the inputting section 3a, while reads out the predetermined PSF correlated to the radiographing conditions from the storage section 3d and also applies the Fourier Transform processing to the predetermined PSF, so as to create the degradation restored image by subtracting the Fourier-Transformed PSF from the Fourier-Transformed X-ray radiological image data.

In this connection, it is preferable to implement the following formula (2) based on the Bayes estimate, instead of simply applying the deconvolution.

$$W_{i,r+1} = W_{i,r} \sum_{k=i}^{c} \frac{S_{k-i+1} H_k}{\sum_{j=a}^{b} S_{k-j+1} W_{j,r}} \quad \cdots (2)$$

a = (l, k - K + 1)max
b = (k, l)min
c = i + K - 1
k = {1, 2, ---K}
i = {1, 2, ---l}

where H:

X ray radiological image
W: degradation restored image
S: Point Spread Function (PSF)
K: number of elements of vector S
I: number of elements of vector W
r: integer equal to or greater than zero, representing a number of repetitions.

Further, the initial value of the formula (2) in the above is represented by the formula (3) indicated as follow.

$$W_{i,1} = \sum_{k=i}^{c} \frac{S_{k-i+1} H_k}{\sum_{j=a}^{b} S_{k-j+1}} \quad \cdots (3)$$

The display section 3f is provided with a display device, so as to display the image represented by the X ray radiological image data after image processing are applied and various kinds of displaying screens on the display device.

Next, the radiological image capturing method, to be implemented in the radiological image capturing system 1, will be detailed in the following.

At first, the user fixes the subject H onto the fixing member 7, so that the values of R1 and R2 fulfill the condition of the formula (1) aforementioned. For instance, when the focusing aperture D = 100 μm and R1 = 0.65 m, 1.0 m, 2.0 m, it is established that R2 ≥ 0.15 m, R2 ≥ 0.10 m, R2 ≥ 0.05 m, respectively corresponding to the value of R1.

Successively, the user installs the plate member 8a, so that the position of the through hole 8b of the plate member 8a coincides with any one of the diagnosis object positions. Still successively, when the user inputs the instruction for commencing the PSF creation processing from the operating section 3b, the control section 3c controls the X ray tube 4 and the image capturing section 5 of the radiographing apparatus 2, so as to radiograph the image of the through hole 8b of the plate member 8a. Then, the image capturing section 5 outputs the acquired reference radiological image data, which are inputted into the image processing apparatus 3 through the inputting section 3a (reference radiological image inputting process). Still successively, the control section 3c creates the luminance distribution from the reference radiological image data inputted from the inputting section 3a, so as to create a function being approximate to a linear shape of the luminance distribution, namely, the Point Spread Function (PSF) at each diagnosis object position of the diagnosis object concerned. Further, the control section 3c stores the created PSF into the storage section 3d as the image restoration parameter.

Still successively, when the X ray tube 4 emits X rays so as to irradiate the emitted X rays onto the subject H under the controlling operations of the control apparatus 6, the image detecting device 5a of the image capturing section 5 detects the irradiated X rays. In this connection, when employing the stimulable phosphor plate as the image detecting device 5a, energy of the X rays are accumulated into the stimulable phosphor plate. Then, the reading section (not shown in the drawings) irradiates the excitation light onto the stimulable phosphor plate so as to electro-photographically convert the stimulated light emitted from the stimulable phosphor plate to image signals, so as to output the generated image signals (analogue signals) to the control apparatus 6. On the other hand, when employing the FPD as the image detecting device 5a, an analogue-to-digital converter (not shown in the drawings) converts the analogue electric signals, generated in the FPD, to digital image data, so as to output the digital image data, acquired by the analogue-to-digital converting operation, to the control apparatus 6.

Still successively, the communication section 6d of the control apparatus 6 transmits the X ray radiological image data acquired in the image capturing section 5 to the image processing apparatus 3. When the X ray radiological image data are inputted from the inputting section 3a of the image processing apparatus 3 through the control section 3c, the storage section 3d stores the X ray radiological image data associated with the radiographing conditions in it.

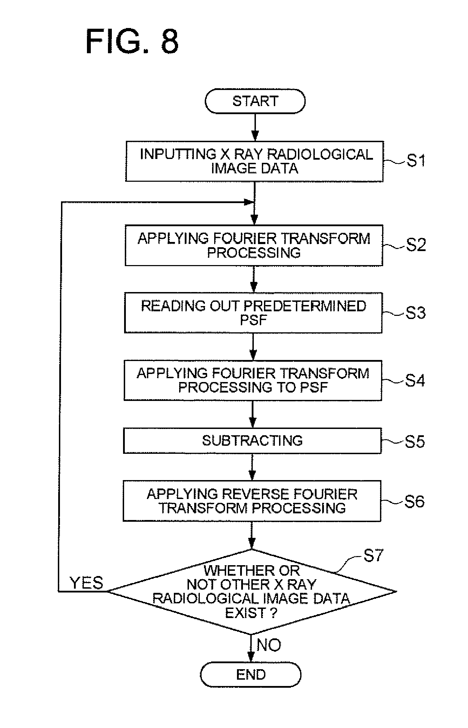

Still successively, the image processing section 3e applies the image processing to the X ray radiological image data. Fig. 8 shows a flowchart indicating the image processing to be conducted by the image processing section 3e. The flowchart of the image processing to be conducted by the image processing section 3e includes the steps of: inputting the X ray radiological image data from the inputting section 3a under the controlling operation of the control section 3c (Step S1); applying the Fourier Transform processing to the X ray radiological image data inputted in the above step (Step S2); reading out the predetermined PSF correlated to the radiographing condition from the storage section 3d (Step S3); applying the Fourier Transform processing to the PSF read out in the above step (Step S4); subtracting the Fourier Transformed PSF processed in Step S4 from the Fourier-Transformed X-ray radiological image data processed in Step S3 (Step S5), so as to generate calculated differential image data; applying the Reverse Fourier Transform processing to the calculated differential image data derived in Step S5 (Step S6), so as to create the degradation restored image data; and returning to Step S2 if the other X ray radiological image data to be processed exist, or finalizing the processing if not (Step S7).

Yet successively, the control section 3c controls the display section 3f so as to display the X ray radiological image represented by the degradation restored image data, generated according to the abovementioned process by the image processing section 3e, on its screen.

Conventionally, when positions of the diagnosis object in a thickness direction of the subject H are different from each other, the point spread distribution of the X rays penetrated through the diagnosis object also varies with the positions. Accordingly, since the image restoration parameter to be employed for restoring the X ray image of each diagnosis object also varies with the positions, it has been impossible to conduct the accurate degraded image restoration processing when only a single image restoration parameter is employed. However, according to the radiological image capturing system 1 and the radiological image capturing method, embodied in the present invention, as described in the foregoing, when the radiographing operation is conducted in such a state that the subject H is fixed at the predetermined position so as to fulfill the formula (1) aforementioned, the ratio of blurs is reduced to a smaller value to such an extent that the difference of blurs in the radiographed images with respect to each diagnosis object can be ignored. Accordingly, irrespective of the position of the diagnosis object in the thickness direction of the subject H, it becomes possible to obtain the accurate X ray radiological image, by applying the degraded image restoration processing, which employs only a single image restoration parameter.

Further, since it is established that the ratio of blurs (= $\delta 1 / \delta 2$) is equal to or smaller than 5.0, under the definition that the point spread distribution of the radiological image $\alpha$, serving as the X ray radiological image detected by the image detecting device 5a and located at the first diagnosis object position of subject H, is $\delta 2$, while the other point spread distribution of the radiological image $\beta$, serving as the other X ray radiological image detected by the image detecting device 5a and located at the second diagnosis object position of subject H, is $\delta 1$, it becomes possible to reduce the difference between the blurs to a smaller value to such an extent that the difference between the blurs can be ignored, and, irrespective of the position of the diagnosis object in the thickness direction of the subject H, it also becomes possible to obtain the accurate X ray radiological image, by applying the degraded image restoration processing, which employs only a single image restoration parameter.

Still further, as far as the gap distance between the first diagnosis object position Pnd the second diagnosis object position is equal to or smaller than 300 mm, it becomes possible to apply the degraded image restoration processing, in which only a single image restoration parameter is employed, to the radiological image concerned without causing any practical problem, irrespective of the position of the diagnosis object in the thickness direction of the subject H.

Yet further, since the image restoration parameter is created on the basis of the reference radiological image of the subject radiographed by the radiographing apparatus 2, it becomes possible to create the restored image corresponding to the individual differences of the X ray tube 4 and/or the image detecting device 5a.

As detailed in the foregoing, according to the radiological image capturing system and the radiological image capturing method, embodied in the present invention, it becomes possible to obtain a radiological image, being more accurate than ever, by employing a simple and easy processing, irrespective of the position of the diagnosis object in the thickness direction of the subject H.

While the preferred embodiments of the present invention have been described using specific term, such description is for illustrative purpose only, and it is to be understood that changes and variations may be made without departing from the spirit and scope of the appended claims.

**Claims**

1.  A radiological image capturing system, comprising:

    a radiographing apparatus that is provided with a radial ray emitting source to emit and irradiate radial rays onto a subject and an image detecting device to detect radial rays penetrated through the subject, so as to output radiological image data based on the radial rays detected by the image detecting device; and
    an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and **characterized in that**
    the radiological image capturing system is further comprises a fixing member to fix the subject onto a predetermined position; and
    formula (1) indicated as follow is fulfilled:

    $$R2 \rangle \frac{A}{R1} \times Log_{10}D \quad \cdots (1)$$

    where R1 (m): distance between the radial ray emitting source and the predetermined position of the subject fixed onto the fixing member,
    R2 (m): distance between the predetermined position of the subject and the image detecting device,
    D ($\mu$m): focusing aperture of the radial ray emitting source, and
    A: constant value equal to or greater than 0.05.

2.  The radiological image capturing system, recited in claim 1, **characterized in that**,
    when a first point spread distribution of first radiological image data, to be detected by the image detecting device at a first diagnosis object position of the subject, is defined as $\delta 1$, while a second point spread distribution of second radiological image data, to be detected by the image detecting device at a second diagnosis object position of the subject, which is apart from the image detecting device by a distance farther than that of the first diagnosis object position, is defined as $\delta 2$, the first point spread distribution $\delta 1$ and the second point spread distribution $\delta 2$ fulfill a relationship indicated as follow:

    $$\delta 1 \ / \ \delta 2 \leq 5 \ .$$

3.  The radiological image capturing system, recited in claim 2, **characterized in that**
    a gap distance between the first diagnosis object position and the second diagnosis object position is equal to or shorter than 300 mm in an irradiating direction of the radial rays.

4.  The radiological image capturing system, recited in any one of claims 1 - 3, **characterized in that**
    the image processing section is further provided with an image restoration parameter creating section to create the image restoration parameter based on reference radiological image data of the subject radiographed by the radiographing apparatus.

5.  A method for capturing radiological image, to be employed in a radiological image capturing system, which includes:
    a radiographing apparatus that is provided with a radial ray emitting source to emit and irradiate radial rays onto a subject and an image detecting device to detect radial rays penetrated through the subject, so as to output radiological image data based on the radial rays detected by the image detecting device; an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and a fixing member to fix the subject onto a predetermined position; the method **characterized by** comprising:

fixing the subject onto the fixing member, so as to position the subject at the predetermined position, which fulfills formula (1) indicated as follow:

$$R2 \rangle \frac{A}{R1} \times Log_{10} D \quad \cdots (1)$$

where R1 (m): distance between the radial ray emitting source and the predetermined position of the subject fixed onto the fixing member,
R2 (m): distance between the predetermined position of the subject and the image detecting device,
D ($\mu$m): focusing aperture of the radial ray emitting source, and
A: constant value equal to or greater than 0.05.

6. The method, recited in claim 5, **characterized in that**,
when a first point spread distribution of first radiological image data, to be detected by the image detecting device at a first diagnosis object position of the subject, is defined as $\delta1$, while a second point spread distribution of second radiological image data, to be detected by the image detecting device at a second diagnosis object position of the subject, which is apart from the image detecting device by a distance farther than that of the first diagnosis object position, is defined as $\delta2$, the first point spread distribution $\delta1$ and the second point spread distribution $\delta2$ fulfill a relationship indicated as follow:

$$\delta1 \; / \; \delta2 \leq 5 \; .$$

7. The method, recited in claim 6, **characterized in that**
a gap distance between the first diagnosis object position and the second diagnosis object position is equal to or shorter than 300 mm in an irradiating direction of the radial rays.

8. The method, recited in any one of claims 5 - 7, **characterized by** further comprising:

creating the image restoration parameter based on reference radiological image data of the subject radiographed by the radiographing apparatus.

9. A radiological image capturing system, comprising:

a radiographing apparatus that is provided with a radial ray emitting source to emit and irradiate radial rays onto a subject and an image detecting device to detect radial rays penetrated through the subject, so as to output radiological image data based on the radial rays detected by the image detecting device; and
an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and **characterized in that**
the radiological image capturing system further comprises a fixing member onto which the subject is fixed; and the fixing member is employed for positioning the subject at such a position that a ratio of blurs between a first radiological image radiographed at a first diagnosis object position of the subject and a second radiological image radiographed at a second diagnosis object position of the subject, which is apart from the image detecting device by a distance farther than that of the first diagnosis object position, is reduced to a value to such an extent that a difference of blurs between the first radiological image and the second radiological image can be ignored.

10. The radiological image capturing system, recited in claim 9, **characterized in that**

the subject is positioned so as to fulfill formula (1) indicated as follow:

$$R2 \rangle \frac{A}{R1} \times Log_{10}D \quad \cdots (1)$$

where R1 (m): distance between the radial ray emitting source and the predetermined position of the subject fixed onto the fixing member,

R2 (m): distance between the predetermined position of the subject and the image detecting device,

D ($\mu$m): focusing aperture of the radial ray emitting source, and

A: constant value equal to or greater than 0.05.

11. The radiological image capturing system, recited in claim 9 or 10, **characterized in that**,

when a first point spread distribution of first radiological image data, to be detected by the image detecting device at the first diagnosis object position of the subject, is defined as $\delta 1$, while a second point spread distribution of second radiological image data, to be detected by the image detecting device at the second diagnosis object position of the subject is defined as $\delta 2$, the first point spread distribution $\delta 1$ and the second point spread distribution $\delta 2$ fulfill a relationship indicated as follow:

$$\delta 1 \: / \: \delta 2 \leq 5 \: .$$

12. The radiological image capturing system, recited in claim 11, **characterized in that**

a gap distance between the first diagnosis object position and the second diagnosis object position is equal to or shorter than 300 mm in an irradiating direction of the radial rays.

13. The radiological image capturing system, recited in any one of claims 9 - 12, **characterized in that**

the image processing section is further provided with an image restoration parameter creating section to create the image restoration parameter based on reference radiological image data of the subject radiographed by the radiographing apparatus.

14. A method for capturing radiological image, to be employed in a radiological image capturing system, which includes: a radiographing apparatus that is provided with a radial ray emitting source to emit and irradiate radial rays onto a subject and an image detecting device to detect radial rays penetrated through the subject, so as to output radiological image data based on the radial rays detected by the image detecting device; an image processing apparatus that is provided with a storage section to store an image restoration parameter established in advance and an image processing section to apply a degraded image restoration processing to the radiological image data outputted from the radiographing apparatus by employing the image restoration parameter read out from the storage section; and a fixing member onto which the subject is fixed; the method **characterized by** comprising:

fixing the subject onto the fixing member, so as to position the subject at such a position that a ratio of blurs between a first radiological image radiographed at a first diagnosis object position of the subject and a second radiological image radiographed at a second diagnosis object position of the subject, which is apart from the image detecting device by a distance farther than that of the first diagnosis object position, is reduced to a value to such an extent that a difference of blurs between the first radiological image and the second radiological image can be ignored.

15. The method, recited in claim 14, **characterized in that**

the subject is positioned so as to fulfill formula (1) indicated as follow:

$$R2 \rangle \frac{A}{R1} \times Log_{10}D \quad \cdots (1)$$

where R1 (m): distance between the radial ray emitting source and the predetermined position of the subject fixed onto the fixing member,

R2 (m): distance between the predetermined position of the subject and the image detecting device,

D ($\mu$m): focusing aperture of the radial ray emitting source, and

A: constant value equal to or greater than 0.05.

**16.** The method, recited in claim 14 or 15, **characterized in that**,

when a first point spread distribution of first radiological image data, to be detected by the image detecting device at a first diagnosis object position of the subject, is defined as $\delta 1$, while a second point spread distribution of second radiological image data, to be detected by the image detecting device at a second diagnosis object position of the subject, which is apart from the image detecting device by a distance farther than that of the first diagnosis object position, is defined as $\delta 2$, the first point spread distribution $\delta 1$ and the second point spread distribution $\delta 2$ fulfill a relationship indicated as follow:

$$\delta 1 \, / \, \delta 2 \leq 5 \, .$$

**17.** The method, recited in claim 16, **characterized in that**

a gap distance between the first diagnosis object position and the second diagnosis object position is equal to or shorter than 300 mm in an irradiating direction of the radial rays.

**18.** The method, recited in any one of claims 14 - 17, **characterized by** further comprising:

creating the image restoration parameter based on reference radiological image data of the subject radiographed by the radiographing apparatus.

FIG. 1

# FIG. 2

POINT SPREAD DISTRIBUTION $\delta 1$

HB

5H

H

Q

4

$\beta$

$D(\mu m)$

X RAY SOURCE

P

$\alpha$

POINT SPREAD DISTRIBUTION $\delta 2$

300mm

10mm

DISTANCE FROM X RAY SOURCE TO SUBJECT R1(m)

DISTANCE FROM SUBJECT TO IMAGE DETECTING DEVICE R2(m)

# FIG. 3

D=100(μm)

RATIO OF BLURS ($\delta 1/\delta 2$)

R1=0.65m

R1=1.0m

R1=2.0m

R2

# FIG. 4

# FIG. 5

# FIG. 6

8b          8a

# FIG. 7

# FIG. 8

```
                    START

        INPUTTING X RAY RADIOLOGICAL      S1
              IMAGE DATA

        APPLYING FOURIER TRANSFORM        S2
              PROCESSING

        READING OUT PREDETERMINED         S3
              PSF

        APPLYING FOURIER TRANSFORM        S4
           PROCESSING TO PSF

              SUBTRACTING                 S5

        APPLYING REVERSE FOURIER          S6
           TRANSFORM PROCESSING

                                          S7
                WHETHER OR
              NOT OTHER X RAY
YES        RADIOLOGICAL IMAGE DATA
                EXIST ?
                                  NO

                    END
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006257762 A
- JP 2509181 B

- JP 3423828 B